# EUROPÄISCHE PATENTSCHRIFT

(11) **EP 0 707 656 B1**
(45) Veröffentlichungstag und Bekanntmachung des Hinweises auf die Patenterteilung: **02.09.1998**
(21) Anmeldenummer: 94919662.0
(22) Anmeldetag: 24.06.1994
(51) Int. Cl.: C12P 7/04, C12P 7/24, C12P 7/40, C12P 17/00, C07D 319/06

(54) **VERFAHREN ZUR BIOTECHNOLOGISCHEN HERSTELLUNG VON ALKOHOLEN, ALDEHYDEN UND CARBONSÄUREN**
BIOTECHNOLOGICAL PROCESS FOR PRODUCING ALCOHOLS, ALDEHYDES AND CARBOXYLIC ACIDS
PROCEDE DE PREPARATION BIOTECHNOLOGIQUE D'ALCOOLS, D'ALDEHYDES ET D'ACIDES CARBOXYLIQUES

(30) Priorität: 05.07.1993 DE 4322276; 31.07.1993 DE 4325850
(43) Veröffentlichungstag der Anmeldung: 24.04.1996
(73) Patentinhaber: BASF AKTIENGESELLSCHAFT, 67056 Ludwigshafen (DE)
(72) Erfinder: PRESSLER, Uwe, D-67122 Altrip (DE); BALKENHOHL, Friedhelm, D-67117 Limburgerhof (DE); HAUER, Bernhard, D-67136 Fussgönheim (DE); LADNER, Wolfgang, D-67136 Fussgönheim (DE); SCHNELL, Ursula, D-33175 Bad Lippsringe (DE); STAUDENMAIER, Horst, Ralf, D-67134 Birkenheide (DE)
(86) Internationale Anmeldenummer: EP9402071
(87) Internationale Veröffentlichungsnummer: WO9502061

(56) Entgegenhaltungen:
- EP-A- 0 442 430
- EP-A- 0 466 042
- EP-A- 0 477 828
- GB-A- 2 149 783
- BIOCHEMICAL JOURNAL, Bd.102, Nr.2, Februar 1967, LONDON Seiten 504 - 510 HOOK G. ET AL. 'Oxidation of Methyl groups by Grass Grubs and Vertebrate Liver Enzymes'
- TETRAHEDRON, (INCL. TETRAHEDRON REPORTS), Bd.48, Nr.32, 7. August 1992, OXFORD GB Seiten 6681 - 6688 ABRAHAM ET AL. 'Microbial Hydroxylation of Activated Acyclic Monoterpene Hydrocarbons' in der Anmeldung erwähnt
- Derwent WPI, Zusammenfassung Nr. 75-03258W [02] & SU-A-0 417 468
- Derwent WPI, Zusammenfassung Nr. 72-23435T [15] & SU-A-0 302 341
- Derwent WPI, Zusammenfassung Nr. 66-40202F [00] & SU-A-0 228 688
- Biocatalysis 1988, 2, 59 - 67
- Eur. Congr. Biotechnol. 1987, 2, 212 - 215

## Beschreibung

Die Erfindung betrifft ein Verfahren zur biotechnologischen Herstellung von Alkoholen, Aldehyden und Carbonsäuren.

Hochfunktionalisierte Alkohole, Aldehyde und Carbonsäuren sind interessante und gesuchte Produkte für die Herstellung von Pharmaka, Polymeren, Feinchemikalien usw., die oft durch Oxidation entsprechender Vorstufen synthetisiert werden.

Chemische Oxidationen zeigen in der Regel geringe Regiospezifitäten. Außerdem werden für die Oxidation oft toxische und/oder schwer handhabbare Reagenzien benötigt.

Von Bakterien ist die Fähigkeit zur Oxidation von Methylgruppen prinzipiell bekannt.

So beschreiben Raymond et al (Appl. Microbiol. 15, 857 - 865 (1967)) die Oxidation von Methylgruppen durch Cooxidation mit n-Paraffinen bei der Gattung Nocardia.

Von der Gattung Pseudomonas ist bekannt, daß sie p-Cymen in drei Schritten über den Alkohol und Aldehyd zur p-Isopropylbenzoesäure oxidiert (J. Bacteriol. 129, 1356 - 1364 und 1365 - 1374 (1977) und ebenda 171, 5155 - 5161 (1989)).

In GB-A-2 149 783 wird die Oxidation von m-Phenoxytoluol zum entsprechenden Alkohol oder Aldehyd mit Hilfe methylotropher, von C1-organischen Verbindungen abhängigen Bakterien beschrieben. Die Oxidation von Methylpyridinen wird in SU-A-D 417 468, SU-A-D 302 341 und SU-A-D 228 688 beschrieben.

Neben dieser Oxidation benzylischer Methylgruppen ist auch die Oxidation allylischer Methylgruppen mit Hilfe einer Reihe von Mikroorganismen bekannt.

So beschreiben Noma et al. die Oxidation von Limonen durch Aspergillus cellulosae (Phytochemistry, 31, 2725 - 2727 (1992)) u.a. zu Perillaalkohol.

Pseudomonas incognita oxidiert Linalool u.a. zu 8-Hydroxylinalool (J. Bacteriol. 171, 5155 - 5161 (1989)).

Bhattacharyya et al. haben in einer ganzen Reihe von Arbeiten (Indian. J. Biochem. 5, 79 - 91 und 92 - 101 (1968), Indian. J. Biochem. 3, 144 - 157 (1966) und Biochem. Biophys. Res. Commun. 29, No. 3, 275 - 279 (1967)) die Oxidation von Terpenen (z.B. Limonen, α- und β-Pinen) durch Pseudomonas PL-Stämme untersucht. Diese PL-Stämme oxidieren auch p-Cymen zur Cuminsäure (Indian. J. Biochem. 5, 161 - 167 (1968)). Die angeführten Arbeiten zeigen jedoch, daß neben den gewünschten Methylgruppenoxidationen in benzylischer und allylischer Position durch die Mikroorganismen eine ganze Reihe zusätzlicher Oxidationen an den eingesetzten Edukten (wie Limonen, p-Cymen etc.) passieren.

Die in CH-PS 677 791 und Tetrahedron 48, 6681 - 6688 (1992) beschriebene Oxidation einer allylischen Methylgruppe von Terpenen mit Mikroorganismen der Gattungen Bacillus, Pseudomonas, Absidia, Rhizopus, Streptomyces, Mycobacterium, Cunninghamella, Nocardia etc. ist ebenfalls mit dem Problem von Nebenreaktionen behaftet.

Die beschriebenen Reaktionen sind deshalb technisch nicht anwendbar.

EP-OS 442 430, EP-OS 466 042 und EP-OS 477 828 beschreiben technisch nutzbare Methylgruppenoxidationen. Nachteil dieser Umsetzungen ist jedoch die Induktion der Mikroorganismen durch z.B. p-Xylol als einziger Kohlenstoff- und Energiequelle. Außerdem sind die beschriebenen Reaktionen auf 5- und 6-gliedrige Heterozyklen beschränkt.

Technische Verfahren, mit Mikroorganismen, die sowohl benzylische als auch allylische Methylgruppenpositionen bei einer breiten Pallette von aromatischen, heteroaromatischen und aliphatischen Substanzen selektiv oxidieren, sind bisher nicht bekannt.

Es wurde nun ein technisch brauchbares Verfahren zur mikrobiellen Herstellung von Alkoholen, Aldehyden und Carbonsäuren gefunden.

Gegenstand der Erfindung ist ein Verfahren zur Herstellung von Verbindungen der Formel I 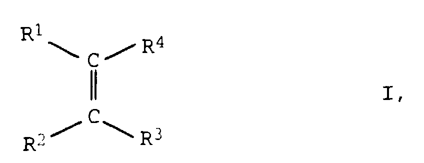 worin
- _{R}1: ein Wasserstoffatom, eine C₁₋₄-Alkyl, C₂₋₄-Alkenyl-oder eine gegebenenfalls substituierte Arylgruppe ist
- R²: ein Wasserstoffatom, eine C₁₋₄-Alkyl-, C₂₋₄-Alkenyl-oder eine gegebenenfalls substituierte Arylgruppe darstellt oder
- R¹ und R²: zusammen mit den Kohlenstoffatomen, an die sie gebunden sind a) Teil eines gegebenenfalls substituierten Ringes oder Ringsystems sind, ausgewählt aus der Gruppe Phenyl, Benzoyl, Naphthyl, Chinolinyl, Isochinolinyl, Benzofuryl oder Benzoxaxyl, wobei der Ring oder das Ringsystem durch folgende Substituenten Alkyl, Alkoxy, Alkenyl, Halogen oder Alkoxycarbonyl, substituiert sein kann, oder b) einen heterocyclischen 5-Ring darstellen, der 1, 2 oder 3 Heteroatome enthält und/oder eine weitere Doppelbindung enthalten kann und an den ein weiterer aromatischer oder heteroaromatischer Ring ankondensiert sein kann, der durch ein bis vier C₁₋₄-Alkoxy-, C₁₋₄-Alkyl-, C₂₋₄-Alkenyl-,Nitril-, Nitro-,Carboxyl-, C₁₋₄-Alkoxycarbonyl-, Amino-, C₁₋₄-Alkylamino-, Di-C₁₋₄-alkylamino- und/oder eine gegebenenfalls substituierte Benzoylgruppe und/oder ein bis vier Halogenatome substituiert sein kann,
- R³: ein Wasserstoff- oder Halogenatom, eine C₁₋₄-Alkyl-oder C₂₋₄-Alkenylgruppe, oder eine gegebenenfalls halogensubstituierte Aryloxy- oder Benzoylgruppe darstellt und
- R⁴: eine -CH₂OH-, oder -CHO- oder -COOH-Gruppe bedeutet,
welches darin besteht, daß man eine Verbindung der Formel II 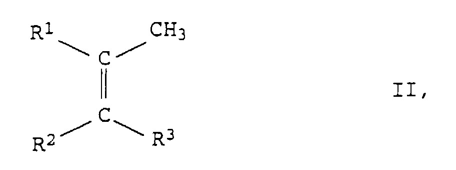 worin R¹-R³ die oben angegebene Bedeutung besitzen, mit Hilfe eines Bakteriums, ausgewählt aus der Gruppe der Gattungen Arthrobacter, Bacillus, Brevibacterium, Corynebacterium, Caseobacter, Gordona, Micrococcus, Mycobacterium, Nocardia, Planococcus, Proactinomyces, Rhodococcus, Staphylococcus, Serratia oder Tsukamurella selektiv die Methylgruppe oxidiert.

Als Alkylreste seien für R¹ und R² genannt: Methyl-, Ethyl-, n-Propyl-, 1-Methylethyl-, Butyl-, 1-Methylpropyl-, 2-Methylpropyl- und 1,1-Dimethylethylgruppen. Bevorzugte Alkylreste sind Methyl-, Ethyl- und Propylreste. Als Alkenylreste sind Vinyl-, 1-Methylvinyl-, cis-1-Butenyl, trans-1-Butenyl-, cis-2-Butenyl, trans-2-Butenyl, 3-Butenyl-, 2-Methyl-1-propenyl-, 2-Methyl-2-propenyl-, 1-Methyl-1-propenyl-, 1-Methyl-2-propenyl-und 1-Ethylvinylreste zu nennen. Bevorzugt ist der 2-Methyl-1-propenyl-Rest.

Ist R¹ eine substituierte Arylgruppe, so trägt diese vorzugsweise C₁₋₄-Alkyl-, C₂₋₄-Alkenyl- und/oder Halogenreste. Als Alkylreste seien genannt: Methyl-, Ethyl-, n-Propyl-, 1-Methylethyl-, Butyl-, 1-Methylpropyl-, 2-Methylpropyl und 1,1-Dimethylethyl-gruppen. Bevorzugte Alkylreste sind Methyl-, Ethyl- und n-Propyl-reste. Als Alkenylreste sind Vinyl-, 1-Methylvinyl-, cis-1-Butenyl, trans-1-Butenyl-, cis-2-Butenyl, trans-2-Butenyl, 3-Butenyl-, 2-Methyl-1-propenyl-, 2-Methyl-2-propenyl-, 1-Methyl-1-propenyl-, 1-Methyl-2-propenyl- und 1-Ethylvinylreste zu nennen. Bevorzugt ist der 2-Methyl-1-propenyl-Rest. Als Halogenatome sind Fluor, Chlor oder Brom zu nennen. Bevorzugt ist Chlor. Die Zahl der Substituenten am Arylrest beträgt normalerweise 1 - 3. Bevorzugt sind 1 oder 2 Reste.

Als Arylreste seien für R¹ besonders der Phenyl- und Naphthylrest genannt.

Ist R² eine substituierte Arylgruppe, so trägt diese vorzugsweise C₁₋₄-Alkyl-, C₂₋₄-Alkenyl- und/oder Halogenreste. Als Alkylreste seien genannt: Methyl-, Ethyl-, n-Propyl-, 1-Methylethyl-, Butyl-, 1-Methylpropyl-, 2-Methylpropyl- und 1,1-Dimethylethyl-gruppen. Bevorzugte Alkylreste sind Methyl-, Ethyl- und Propylreste. Als Alkenylreste sind Vinyl-, 1-Methylvinyl-, cis-1-Butenyl, trans-1-Butenyl-, cis-2-Butenyl, trans-2-Butenyl, 3-Butenyl-, 2-Methyl-1-propenyl-, 2-Methyl-2-propenyl-, 1-Methyl-1-propenyl-, 1-Methyl-2-propenyl- und 1-Ethylvinylreste zu nennen. Bevorzugt ist der 2-Methyl-1-propenyl-Rest. Als Halogenatome sind Fluor, Chlor oder Brom zu nennen. Bevorzugt ist Chlor. Die Zahl der Substituenten am Arylrest beträgt normalerweise 1 - 3. Bevorzugt sind 1 oder 2 Reste.

Bevorzugte Reste für R² sind: Phenyl, Methyl, p-Methoxyphenyl, 1,3-Dioxanyl und 5,5-Dimethyl-1,3-dioxanyl.

Als Arylreste seien für R² besonders der Phenyl- und Naphthylrest genannt.

Sind R¹ und R² Teil eines Ringes bzw. Ringsystems, so sind folgende als bevorzugt zu nennen: Phenyl, Benzoyl, Naphthyl, Chinolinyl, Isochinolinyl, Benzofuryl und Benzoxazyl.

Als Substituenten dieser Ringsysteme sind zu nennen:

Alkyl wie Methyl, Ethyl, Propyl, 1-Methylethyl, Butyl, 1-Methylpropyl, 2-Methylpropyl, 1,1-Dimethylethyl (insbesondere Methyl, Ethyl und Propyl); Alkoxy wie Methoxy, Ethoxy, Propyloxy, 1-Methylethoxy, Butyloxy, 1-Methylpropyloxy, 2-Methylpropyloxy, 1,1-Dimethylethoxy (insbesondere Methoxy und 1-Methylethoxy); Alkenyl wie Vinyl, 1-Methylvinyl, cis-1-Propenyl, trans-1-Propenyl, 2-Propenyl, cis-1-Butenyl, trans-1-Butenyl, cis-2-Butenyl, trans-2-Butenyl, 3-Butenyl, 2-Methyl-1-propenyl, 2-Methyl-2-propenyl, 1-Methyl-1-propenyl, 1-Methyl-2-propenyl, 1-Ethylvinyl (insbesondere 2-Methyl-1-propenyl); Halogen wie Fluor, Chlor, Brom und Iod (insbesondere Fluor und Chlor); Alkoxycarbonyl wie Methoxycarbonyl, Ethoxycarbonyl, Propyloxy-carbonyl, l-Methylethoxycarbonyl, Butyloxycarbonyl und 1,1-Dimethylethoxycarbonyl (insbesondere Methoxycarbonyl und 1-Methylethoxycarbonyl).

Die Zahl der Substituenten am Ring beträgt normalerweise 1 - 3, vorzugsweise 1 oder 2.

Als C₁₋₄-Alkylreste an der Aminogruppe in den Ringsystemen in R¹ und R² seien genannt : Ethyl, Propyl, 1-Methylethyl, Butyl, 1-Methylpropyl, 2-Methylpropyl, 1,1-Dimethylethyl und insbesondere Methyl.

Für R³ seien folgende Reste speziell genannt: Methyl, Ethyl, n-Propyl, 1-Methylethyl, Butyl, 1-Methylpropyl, 2-Methylpropyl, 1,1-Dimethylethyl, Vinyl, 1-Methylvinyl, cis-1-Propenyl, trans-1-Propenyl, 2-Propenyl, cis-1-Butenyl, trans-1-Butenyl, cis-2-Butenyl, trans-2-Butenyl, 3-Butenyl, 2-Methyl-1-propenyl, 2-Methyl-2-propenyl, 1-Methyl-1-propenyl, 1-Methyl-2-propenyl, 1-Ethylvinyl, Fluor, Chlor und Brom. Bevorzugt für R³ sind: Methyl, Ethyl, n-Propyl und 2-Methyl-1-propenyl.

Das neue Verfahren eignet sich besonders gut zur Herstellung von Alkoholen und insbesondere Carbonsäuren.

Durch Screening nach der Vorschrift von Drews (Mikrobiologisches Praktikum, 3. Auflage, Springer Verlag, Seite 47 bis 48, 1976) lassen sich aus Bodenproben und Stammsammlungen Bakterien isolieren bzw. identifizieren, die die erfindungsgemäße Oxidation durchführen.

Für die erfindungsgemäße Reaktion eignen sich beispielsweise Bakterien, die in der Vergangenheit unter den Gattungsnamen Arthrobacter, Bacillus, Brevibacterium, Corynebacterium, Caseobacter, Gordona, Micrococcus, Mycobacterium, Nocardia, Planococcus, Proactinomyces, Rhodococcus, Staphylococcus, Serratia und Tsukamurella und speziell unter den Gattungs- und Artnamen Arthrobacter sp., Bacillus rubropertinctus, Bacillus mycoides roseus, Bacillus rubricus, Corynebacterium rubrum, Gordona lentifragmenta, Gordona rubropertinctus, Gordona rubra, Micrococcus roseus, Micrococcus roseus roseofulvus, Micrococcus tetragenus ruber, Micrococcus rubens, Micrococcus flavoroseus, Micrococcus corallinus, Micrococcus agilis, Mycobacterium rubropertinctus, Nocardia corallina, Nocardia rubra, Nocardia sp., Nocardia salmonicolor, Nocardia pellegrino, Proactinomyces ruber, Proactinomyces rubropertinctus, Rhodococcus ruber, Rhodococcus rhodochrous, Rhodococcus erythropolis, Rhodococcus rubra, Rhodococcus rubrus, Rhodococcus sp., Rhodococcus roseus, Rhodococcus lentifragmentus, Rhodococcus agilis, Serratia rubropertincta und Staphylococcus roseus geführt wurden.

Die Vielzahl der für die Reaktion in Frage kommenden Bakterien spiegelt die sehr wechselvolle taxonomische Historie der Familien Mycobacteriaceae, Nocardiaceae und Micrococcaceae mit ihren Gattungen Corynebacterium, Gordona, Mycobacterium, Nocardia, Rhodococcus, Tsukamurella, Micrococcus, Staphylococcus und Planococcus wieder.

Näheres ist The Prokaryotes (ed. Balows A. et al) 1992, Vol II., Bergey's Manual of Determinative Bacteriology, Eighth Edition (ed. Cowan S. et al) 1974, Bergey's Manual of Systematic Bacteriology (ed. Seneath, P.H.A. et al.) 1986, Vol II, ATCC-Catalogue of Bacteria & Bacteriophages 17^{th} Edition 1989, Kocur, M. et al, Int. J. Syst. Bacteriol. 20, 233 - 240 (1970), und Tsukamura, M. et al, Int. J. Syst. Bacteriol. 25, 377 - 382 (1975) und J. Gen. Microbiol. 68, 15 - 26 (1971), 80, 553 - 555 (1974) und 125, 205 - 208 (1981) zu entnehmen.

Die taxonomische Stellung der aufgeführten Gattungen unterlag in den letzten Jahren einem starken Wandel und befindet sich noch immer im Fluß, da falsche Gattungs- und Artnamen korrigiert werden und bestehende Stämme in neue Gattungen sortiert werden. Innerhalb dieser Gattungen und Arten bestehen enge verwandtschaftliche Beziehungen. Bakterien, die die erfindungsgemäße Reaktion durchführen, sind mit einer guten Wahrscheinlichkeit in den oben genannten Gattungen und Arten zu finden.

Besonders geeignet für die Methylgruppenoxidation sind folgende Stämme:

Rhodococcus ruber (Nocardia pellegrino) DSM 43232, Rhodococcus ruber (Rhodococcus rubrus, Nocardia sp.) DSM 43250, Rhodococcus ruber (Rhodococcus rubrus, Nocardia sp.) DSM 43251, Rhodococcus ruber (Rhodococcus rubrus, Nocardia sp.) DSM 43252, Rhodococcus ruber (Rhodococcus rubrus, Nocardia sp.) DSM 43335, Rhodococcus ruber (Rhodococcus rubrus, Nocardia sp.) DSM 43338, Rhodococcus ruber (Nocardia pellegrino) IMET 7308, Rhodococcus ruber IMET 7337, Rhodococcus ruber (Rhodococcus lentifragmentus, Gordona lentifragmenta) IMET 7437 und Rhodococcus ruber (Gordona lentifragmenta) IMET 7477, Rhodococcus ruber (Nocardia rubra) LMG 5366, Micrococcus roseus (Micrococcus roseus roseofulvus, Micrococcus tetragenus ruber) ATCC 178, Micrococcus roseus (Micrococcus rubens, Micrococcus roseus roseofulvus, Micrococcus tetragenus ruber) ATCC 186, Micrococcus roseus (Rhodococcus roseus) ATCC 516, Micrococcus roseus (Rhodococcus ruber) ATCC 534 und Micrococcus roseus ATCC 9815.

Die Angaben in den Klammern geben die frühere Stammbezeichnungen wieder.

Ganz besonders eignet sich für die Oxidation der Stamm Rhodococcus ruber DSM 8316, sowie sich davon ableitende Mutanten. Dieser Stamm ist durch die in der Anlage ermittelten Daten charakterisiert.

Bei Rhodococcus ruber DSM 8316 handelt es sich um ein Isolat aus Bodenproben.

Die Methylgruppe muß, um zum entsprechenden Alkohol, Aldehyd oder Carbonsäure oxidierbar zu sein, benzyl- oder allylständig sein. Sind mehrere benzyl- oder allylständige Methylgruppen im Edukt vorhanden, so wird in der Regel nur eine Methylgruppe selektiv oxidiert. In seltenen Fällen (< 5 %) wird eine weitere Methylgruppe oxidiert.

Da die Edukte über die Sequenz Alkohol, Aldehyd und Carbonsäure abgebaut werden und den aufgeführten Wildtypstämmen als Kohlenstoff- und Energiequelle dienen, ist es zweckmäßig, zur Gewinnung der gewünschten Produkte mit Blockmutanten der aufgeführten Bakterien zu arbeiten, die die Oxidation nur bis zum Alkohol, oder nur zum Aldehyd oder nur zur Carbonsäure führen.

Zur Erzeugung solcher Blockmutanten können bekannte mikrobiologische Techniken eingesetzt werden. Zur Auslösung von Mutationen können alle gängigen Methoden verwendet werden wie die Anwendung von mutagenen Substanzen, z.B. Nitrosoguanidine, Ethylmethansulfonat, Natriumnitrit, oder die Einwirkung von elektromagnetischer Strahlung wie UV-, Gamma- oder Röntgenstrahlung. Eine entsprechende Vorschrift zur Herstellung von N-Methyl-N'-nitrosoguanidin ist Biochem. Biophys. Res. Commun. 18, 788 (1965) zu entnehmen. Weiterhin können zur Mutagenese auch transponierbare genetische Elemente verwendet werden.

Zur Isolierung der Mutanten kann beispielsweise die Eigenschaft, auf den Produkten der Oxidation als einziger C-Quelle nicht mehr zu wachsen, benutzt werden.

Das Arbeiten mit solchen Blockmutanten ist eine bevorzugte Ausführungsform der Erfindung.

Das erfindungsgemäße Verfahren zur Oxidation benzylischer und allylischer Methylgruppen an kondensierten und nichtkondensierten Aromaten, Heteroaromaten sowie Aliphaten wird zweckmäßig derart durchgeführt, daß die Bakterien oder deren Mutanten in einem geeigneten Medium mit einer geeigneten Kohlenstoff- und Energiequelle in Gegenwart der entsprechenden Edukte kultiviert werden oder die Edukte nach 24 bis 72 h Kultivierung der Nährlösung zugegeben werden. Schwerlösliche Edukte werden zweckmäßig mit einem handelsüblichen Emulgator (z.B. Tween^{®} 80) als Emulsion dem Nährmedium zugegeben.

Die Fermentation erfolgt kontinuierlich oder diskontinuierlich für 1 bis 14 Tage.

In der Regel wird die Oxidation mit aktiven Bakterien durchgeführt, sie gelingt jedoch auch mit ruhenden Bakterien, allerdings mit wesentlich geringerer Geschwindigkeit.

Geeignet sind Nährmedien, die Kohlenstoffquellen, Stickstoffquellen, anorganische Salze und gegebenenfalls geringe Mengen an Spurenelementen und Vitaminen enthalten. Als Stickstoffquellen können anorganische bzw. organische Stickstoffverbindungen oder Materialien, die diese Verbindungen enthalten, verwendet werden. Beispiele sind Ammoniumsalze, Nitrate, Maisquellwasser, Bierhefeautolysat, Sojabohnenmehl, Weizengluten, Hefeextrakt, Hefe, Harnstoff und Kartoffelprotein.

Als Kohlenstoffquellen können beispielsweise Zucker wie Glucose, Polyole wie Glycerin oder Fette wie Sojaöl verwendet werden.

Beispiele für anorganische Salze sind die Salze von Calcium, Magnesium, Mangan, Kalium, Zink, Kupfer und Eisen. Als Anion der Salze ist besonders das Phosphation zu nennen.

Gegebenenfalls werden dem Nährmedium Wachstumsfaktoren zugesetzt, wie z.B. Biotin, Riboflavin und/oder andere Vitamine.

Das Mischungsverhältnis der genannten Nährstoffe hängt von der Art der Fermentation ab und wird im Einzelfall festgelegt.

Im allgemeinen eignen sich zur Durchführung des erfindungsgemäßen Verfahrens Eduktkonzentrationen von etwa 1 bis 100 g/l, bevorzugt sind Konzentrationen von etwa 3 bis 50 g/l, besonders bevorzugt sind Konzentrationen von etwa 5 bis 20 g/l.

Die Züchtungsbedingungen werden so festgelegt, daß die bestmöglichen Ausbeuten erreicht werden. Bevorzugte Züchtungstemperaturen liegen bei 15°C bis 40°C. Besonders vorteilhaft sind Temperaturen zwischen 25°C und 35°C. Vorzugsweise wird der pH-Wert in einem Bereich von 3 bis 9 festgehalten. Besonders vorteilhaft sind pH-Werte zwischen 5 und 8. Im allgemeinen ist eine Inkubationsdauer von 15 bis 120 Stunden ausreichend. Innerhalb dieser Zeit reichert sich die maximale Menge des gewünschten Produktes im Medium an.

Der Reaktionsumsatz kann leicht durch Probenentnahme und deren Untersuchung durch beispielsweise Gaschromatographie oder mittels HPLC-Analyse verfolgt und kontrolliert werden. Die Isolierung und Reinigung der Produkte aus der Kulturflüssigkeit kann nach bekannten Methoden erfolgen. Zweckmäßigerweise trennt man die feste Biomasse vom Nährmedium ab, extrahiert den Wertstoff z.B. mit einem organischen Lösungsmittel, gegebenenfalls nach vorausgegangener Ansäuerung des Mediums, und isoliert den Wertstoff aus der extrahierten Phase. Auch säulenchromatographische Verfahren sind für die Aufarbeitung zweckmäßig.

### Beispiele

Die folgenden Beispiele erläutern die Erfindung.

Für die angeführten Beispiele wurden zwei Medien benutzt:

| Medium A (Komplexmedium) | |
|---|---|
| 10,0 g/l | Hefeextrakt |
| 20,0 g/l | Glucose |
| 0,5 g/l | Magnesiumsulfat-7-hydrat |
| 1,5 g/l | Kaliumdihydrogenphosphat |
| 3,6 g/l | Dikaliumhydrogenphosphat |
| 2 mg/l | Eisen(II) sulfat-1-hydrat |
| 5 mg/l | ^{®}Titriplex III |
| 100 µg/l | Zink(II)sulfat-4-hydrat |
| 300 µg/ | Borsäure |
| 200 µg/l | Cobalt (II)chlorid-6-hydrat |
| 10 µg/l | Kupfer (II) chlorid-2-hydrat |
| 20 µg/l | Nickel(II)chlorid-6-hydrat |
| 30 µg/l | Natriummolybdat-2-hydrat |

| Medium B (Minimalmedium) | |
|---|---|
| 5,0 g/l | Glucose |
| 2,0 g/l | Kaliumdihydrogenphosphat |
| 0,5 g/l | Magnesiumsulfat-7-hydrat |
| 0,2 g/l | Calciumchlorid-7-hydrat |
| 2,0 g/ | Natriumchlorid |
| 0,5 g/l | Harnstoff |
| 2 mg/l | Eisen(II)sulfat-1-hydrat |
| 5 mg/l | ^{®}Titriplex III |
| 100 µg/l | Zink(II)sulfat-4-hydrat |
| 300 µg/l | Borsäure |
| 200 µg/l | Cobalt (II)chlorid-6-hydrat |
| 10 µg/l | Kupfer (II) chlorid-2-hydrat |
| 20 µg/l | Nickel(II)chlorid-6-hydrat |
| 30 µg/l | Natriummolybdat-2-hydrat |
| 2 µg/l | Biotin |
| 2 µg/l | Folsäure |
| 200 µg/l | p-Aminobenzoesäure |
| 200 µg/l | Riboflavin |
| 400 µg/l | Ca-Pantothenat |
| 1400 µg/l | Nicotinsäure |
| 400 µg/l | Pyridoxin/HCl |
| 2000 µg/l | Meso-Inosit |
| 400 µg/l | Thiamin/HCl |

Der pH-Wert des Mediums wurde mit 5 N Natronlauge bzw. Kalilauge auf 6,8 eingestellt. Glucose und Phosphat wurden jeweils getrennt bei 121°C für 20 min autoklaviert. Harnstoff, Vitamine sowie die entsprechenden Edukte wurden sterilfiltriert. Edukte, die nicht filtrierbar waren, wurden als autosteril angenommen. Das restliche Medium wurde bei 121°C 20 min autoklaviert.

### Beispiel 1

Jeweils 20 ml des entsprechenden Mediums wurden in sterile 100 ml Erlenmeyerkolben gefüllt, die mit einem sterilen Watteverschluß versehen waren. Die Kulturbrühe wurde jeweils mit einer Impföse von DSM 8316 angeimpft. Die in der Tabelle 1 aufgeführten Edukte wurden in einer Konzentration von 5 g/l im Medium B eingesetzt. Die Kolben wurden bei 28°C und 150 Umdrehungen pro min (UpM) für drei bis zehn Tage schüttelnd inkubiert.

Anschließend wurden die Kulturbrühen (siehe Tabelle 1 und 3) aufgearbeitet und analysiert (vgl. Beispiel 4). Die Produkte wurden mit GC, GC/MS und ¹H-NMR identifiziert. Als Referenz dienten authentische, chemisch synthetisierte Proben.

**Tabelle 1**

| Biotransformation mit DSM 8316 - Edukte und analysierte Produkte | |
|---|---|
| Edukt | Produkt |
| 4-Isopropyltoluol | 4-Isopropylbenzoesäure |
| 2-(4-Methylphenoxy-)propan | 4-Isopropoxybenzoesäure |
| 4-Methylanisol | 4-Methoxybenzoesäure |
| 4-Methylacetophenon | 4-Acetylbenzoesäure |
| 4-Cyanotoluol | 4-Cyanobenzoesäure |
| 4-Chlortoluol | 4-Chlorbenzoesäure |
| 4-Vinyltoluol | 4-Vinylbenzoesäure |
| 4-Nitrotoluol | 4-Nitrobenzoesäure |
| 2,3-Dimethyl-4-nitrobenzol | 2-Methyl-4-nitrobenzoesäure |
| 2-Chlor-4-methylnitrobenzol | 2-Chlor-4-nitrobenzoesäure |
| 2,4-Dimethylbenzophenon | 2-Methyl-4-benzoylbenzoesäure |
| 2-Methylnaphthalin | 2-Naphthoesäure |
| 2,6-Dimethylnaphthalin | 6-Methyl-2-naphthoesäure |
| 6-Methylchinolin | 6-Chinolincarbonsäure |
| 2,6-Dimethylchinolin | 2-Methyl-6-chinolincarbonsäure |
| 6-Methylisochinolin | 6-Isochinolincarbonsäure |
| p-Xylol | p-Tolylsäure |
| 4-tert-Butyltoluol | 4-tert-Butylbenzoesäure |
| 2-Methylindol | 2-Indolylcarbonsäure |
| 2-Methylbenzoxazol | 2-Benzoxazolcarbonsäure |
| 2-Methylbenzofuran | Benzofuran-2-carbonsäure |
| 4(Methyl-1-propenyl-)anisol | 4-Methoxy-α-methylzimtsäure |
| Limonen | Perillasäure |
| 2- (2-Methyl-1-propenyl)-5,5-dimethyl-1,3-dioxan | 3-Formyl-2-methyl-2-propensäure(2,2-dimethylpropylen)acetal |

Das 3-Formyl-2-methyl-2-propensäure(2,2-dimethylpropylen)acetal (Formel III) ist eine neue Verbindung, die sich gut zur Herstellung von Polyenen und Carotinoiden eignet.

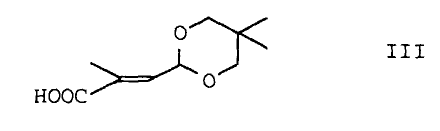

### Beispiel 2

Analog Beispiel 1 wurden die in Tabelle 2 aufgeführten Mikroorganismen in Medium A angezogen. Dem Medium waren jeweils 5 g/l der in Tabelle 3 genannten Edukte zugesetzt worden. Tabelle 3 sind ebenfalls die analog Beispiel 1 nachgewiesenen Produkte zu entnehmen.

**Tabelle 2**

| Mikroorganismen | |
|---|---|
| Rhodococcus ruber | DSM 43250, DSM 43251, DSM 43252, DSM 43232, DSM 43335 und DSM 43338 |
| | |
| Rhodococcus ruber | IMET 7308, IMET 7337, IMET 7437 und IMET 7477 |
| | |
| Rhodococcus ruber | LMG 5366 |
| | |
| Micrococcus roseus | ATCC 178, ATCC 186, ATCC 516, ATCC 534 und ATCC 9815 |

**Tabelle 3**

| Biotransformation mit unter Tabelle 2 aufgeführten Mikroorganismen - Edukte und analysierte Produkte | |
|---|---|
| Edukt | Produkt |
| 2- (4-Methylphenoxy-)propan | 4-Isopropoxybenzoesäure |
| 4-Cyanotoluol | 4-Cyanobenzoesäure |
| 4-Chlortoluol | 4-Chlorbenzoesäure |
| 2-Methylbenzofuran | Benzofuran-2-carbonsäure |
| Limonen | Perillasäure |
| 2-(2-Methyl-1-propenyl)-5,5-dimethyl-1,3-dioxan | 3-Formyl-2-methyl-2-propensäure-(2,2-dimethylpropylen)-acetal |

### Beispiel 3

Analog Beispiel 1 wurde DSM 8316 in einen 500 ml Erlenmeyerkolben in 100 ml Medium A gezüchtet. Dem Medium wurde 5 g/l 2-(4-Methyl-phenoxy-)propan zugegeben. Nach 24 h und 48 h Inkubation wurden nochmals 2,5 g/l Edukt zugegeben. Nach 120 h Inkubation wurde die Kulturbrühe aufgearbeitet. Als Produkte ließen sich neben 4-Isopropoxybenzoesäure als Hauptprodukt in geringen Mengen 4-Isopropoxybenzaldehyd und 4-Isopropoxybenzylalkohol identifizieren. Die Identifizierung der Produkte erfolgte wie unter Beispiel 1 beschrieben.

### Beispiel 4

### a) Herstellung von Blockmutanten

Ausgehend von DSM 8316 wurden, wie in Biochem. Biophys. Res. Commun. 18, 788 (1965) beschrieben, Mutanten selektioniert. Dazu wurde DSM 8316 in 20 ml Medium B über Nacht (30°C, 120 Upm, 16 h) gezüchtet. Anschließend wurden die Zellen geerntet (Zentrifugation: 10 min, 4°C, 5000 g) und zweimal mit 100 mM Tris/HCl-Puffer (pH 7,2) gewaschen. Die so gewonnenen Zellen wurden mit dem Puffer auf eine optische Dichte bei 600 nm von 0,75 eingestellt. 0,5 ml dieser Zellsuspension wurden durch Zugabe von 1 % N-Methyl-N-nitro-N-nitrosoguanidin in DMF in Gegenwart von 100 mM Trismaleinsäurepuffer (pH 6,0) mutagenisiert (30 min, Raumtemperatur, 120 Upm). Das Volumen des Ansatzes betrug 5 ml.

Nach Mutagenese wurden die Zellen zweimal mit 100 mM Tris/HCl-Puffer (pH 7,2) gewaschen und eine Verdünnungsreihe in 10er Schritten bis auf 10⁻⁷ auf Medium B-Agar (= Medium B + 20 g/l Agar) ausplattiert (= Masterplatten). Nach 4 Tagen Inkubation bei RT wurden die entstandenen Kolonien zur Selektion auf Medium B-Agar-Platten (= "replica-Platten") ohne Glucose mit den entsprechenden Edukten und Produkten als einziger C-Quelle (0,5 g/l), wie in Microbiology, Third Edition, Harper International Edition, 1980 beschrieben, gestempelt. Durch Vergleich der replica-Platten mit den Masterplatten konnten Blockmutanten identifiziert werden. Selektionskriterium für die Blockmutanten war das mangelnde Wachstum auf den Edukten und Produkten als einziger C-Quelle.

Eine der so gewonnenen Blockmutanten (= 4a1) wurde in 100 ml Medium A in Gegenwart von 5 g/l 2-(4-Methylphenoxy)-propan, wie in Beispiel 1 beschrieben, gezüchtet. Nach 7 Tagen Inkubation wurden 1000 µl Fermentationsbrühe entnommen und mit 100 µl 1 N HCl und 500 µl MTB (Methyltertiärbutylether) versetzt und 1 min kräftig durchmischt. 400 µl der organischen Phase wurden vorsichtig abgenommen und unter Vakuum eingeengt. Der Rückstand wurde in 100 µl MTB aufgenommen und quantitativ in ein Probengläschen für die Gaschromatographie überführt. Zur Probe wurden 50 µl N-Methyl-N-trimethylsilyltrifluoracetamid gegeben. Als einziges Produkt wurde gaschromatographisch 4-Isopropoxybenzoesäure nachgewiesen. Dies wurde durch GC/MS, ¹H-NMR und Vergleich mit einer authentischen Referenzprobe bestätigt.

Wiederholt man die Inkubation mit einer anderen Blockmutanten (4a2 und 4a3), so kann man im Fall der Mutante (4a2) 4-Isopropoxybenzylalkohol und im Fall der Mutante (4a3) 4-Isopropoxybenzaldehyd als einziges Produkt nachweisen.

### Beispiel 5

### b) Herstellung von 4-Isopropoxy-benzoesäure.

100 ml Medium A wurden mit einer Impföse der Blockmutante 4a1 angeimpft und 48 h inkubiert. Mit 50 ml dieser Kultur wurde ein Laborfermenter mit 1 l Medium A und 5 g 2-(4-Methylphenoxy-)propan als Edukt beimpft. Nach 24 h und 48 h Inkubation wurden nochmals je 2,5 g 2-(4-Methylphenoxy-)propan zugegeben. Nach 168 h Inkubation wurde die Fermentation abgebrochen. Zunächst wurde die Biomasse durch Zentrifugation (30 min, 4°C, 10.000 g) entfernt und die restlichen Zellen durch Filtration abgetrennt. Anschließend wurde die Kulturbrühe mit konzentrierter H₂SO₄ auf pH 1,0 eingestellt und zweimal mit je 250 ml MTB extrahiert. Die organische Phase wurde abgenommen und im Rotationsverdampfer eingeengt. In chemisch reiner Form konnten 7,1 g 4-Isopropoxy-benzoesäure isoliert werden. Dies entspricht einer Ausbeute von etwa 60 %.

### Beispiel 6

### c) Herstellung von 4-Isopropoxybenzylalkohol

100 ml Medium A wurden mit einer Impföse der Blockmutante 4a2 angeimpft und 48 h bei 30°C und 120 Upm inkubiert. Mit 50 ml dieser Kultur wurde ein 1 l Laborfermenter mit Medium A und 5 g 2-(4-Methylphenoxy-)propan als Edukt beimpft. Nach 168 h Inkubation wurde die Fermentation abgebrochen. Zunächst wurde die Biomasse durch Zentrifugation (30 min, 4°C, 10000 x g) entfernt und die restlichen Zellen durch Filtration abgetrennt. Anschließend wurde die Kulturbrühe zweimal mit je 250 ml MTB extrahiert. Die organische Phase wurde abgenommen (MTB-Phase 1). Nach Zugabe von konzentrierter H₂SO₄ bis zu einem pH von 1,0 wurde nochmals zweimal mit je 250 ml MTB nachextrahiert. Die organische Phase wurde wieder abgenommen (MTB-Phase 2). Die organischen Phasen wurden vereinigt und im Rotationsverdampfer eingeengt und analysiert (siehe Beispiel 1). In chemisch reiner Form konnten 942 mg 4-Isopropoxybenzylalkohol als einziges Produkt isoliert werden. Dies entspricht einer Ausbeute von etwa 20 %.

Beschreibung des Mikroorganismus DSM 8316:

Der Stamm DSM 8316 wurde bei der DSM (Deutsche Sammlung von Mikroorganismen und Zellkulturen GmbH, Braunschweig) als Rhodococcus ruber identifiziert. Das Bestimmungsprotokoll lautet wie folgt:
1. Farbtyp: 70 pastellorange bis reinorange
2. Peptidoglycan: meso-Diaminopimelinsäure
3. Mycolsäuren: Kettenlänge von C₃₀-C₅₀
4. Fettsäuremuster: unverzweigte gesättigte und ungesättigte Fettsäuren plus Tuberculostearinsäure. Diess Fettsäuremuster ist diagnostisch für Rhodococcen und ihre Verwandten.
5. Menachinone: MK-8 (H₂)
6. Test 35 weiterer Substrate

| Name | Gesamt P | Willcox P | Tax Distanz | Std.Fehler |
|---|---|---|---|---|
| Rh. ruber | 3.0E-0014 | 78.05 % | 0.495860003 | -4.451097 |
| Rh. marinonascens 43752T | 7.5E-0015 | 19.69 % | 0.442831797 | 1.096741 |
| Rh. rhodochrous | 4.7E-0016 | 1.22 % | 0.509506764 | -4.942624 |
| Rh. coprophilus | 4.0E-0016 | 1.03 % | 0.477056451 | -2.850872 |

| Testergebnisse: | | | |
|---|---|---|---|
| nag 1 - | gal 40 - | gct 1 - | got 20 - |
| gat 1 - | rha 60 - | rib 1 - | suc 40 - |
| tur 1 - | cap 1 - | ara 99 + | ino 20 - |
| cit 80 + | o2V 1 - | o2g 20 + | pim 99 - |
| sat 80 - | ala 20 - | a4b 80 - | asp 6 - |
| leu 80 - | pro 1 - | ser 1 - | val 80 - |
| put 99 - | tyr 1 - | ata 99 - | ben 80 - |
| o3b 99 + | o4b 99 + | phe 1 + | qui 99 + |
| cxy 1 - | cch 99 + | cdp 99 + | |
| Testanzahl: 35/35 Berechnungs-Funktion: 99/1 Cluster aus 16 getesteten Stämmen | | | |

DSM 8316 ist ein Rhodococcus ruber Stamm mit einer Willcox Probability von 90 %.

## Patentansprüche

1. Verfahren zur Herstellung von Verbindungen der Formel I 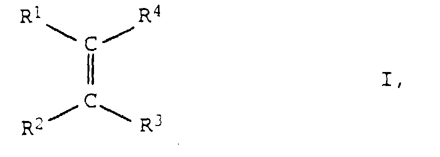 worin
R¹ ein Wasserstoffatom, eine C₁₋₄-Alkyl-, C₂₋₄-Alkenyl-oder eine gegebenenfalls substituierte Arylgruppe ist
R² ein Wasserstoffatom, eine C₁₋₄-Alkyl-, C₂₋₄-Alkenyl-oder eine gegebenenfalls substituierte Arylgruppe darstellt oder
R¹ und R² zusammen mit den Kohlenstoffatomen, an die sie gebunden sind a) Teil eines gegebenenfalls substituierten Ringes oder Ringsystems sind, ausgewählt aus der Gruppe Phenyl, Benzoyl, Naphthyl, Chinolinyl, Isochinolinyl, Benzofuryl oder Benzoxazyl, wobei der Ring oder das Ringsystem durch folgende Substituenten Alkyl-, Alkoxy-, Alkenyl-, Halogen oder Alkoxycarbonyl- substituiert sein kann oder b) einen heterocyclischen 5-Ring darstellen, der 1, 2 oder 3 Heteroatome enthält und/oder eine weitere Doppelbindung enthalten kann und an den ein weiterer aromatischer oder heteroaromatischer Ring ankondensiert sein kann, der durch ein bis vier C₁₋₄-Alkoxy-, C₁₋₄-Alkyl-, C₂₋₄-Alkenyl-, Nitril-, Nitro-, Carboxyl-, C₁₋₄-Alkoxycarbonyl-, Amino-, C₁₋₄-Alkylamino-, Di-C₁₋₄-alkylamino- und/oder eine gegebenenfalls substituierte Benzoylgruppe und/oder ein bis vier Halogenatome substituiert sein kann,
R³ ein Wasserstoff- oder Halogenatom, eine C₁₋₄-Alkyl-oder C₂₋₄-Alkenylgruppe, oder eine gegebenenfalls halogensubstituierte Aryloxy- oder Benzoylgruppe darstellt und
R⁴ eine -CH₂OH-, oder -CHO- oder -COOH-Gruppe bedeutet,
dadurch gekennzeichnet, daß man in Verbindungen der Formel II 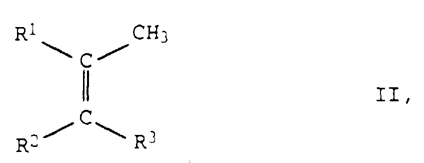 worin R¹-R³ die oben angegebene Bedeutung besitzen, mit Hilfe eines Bakteriums ausgewählt aus der Gruppe der Gattungen Arthrobacter, Bacillus, Brevibacterium, Corynebacterium, Caseobacter, Gordona, Micrococcus, Mycobacterium, Nocardia, Planococcus, Proactinomyces, Rhodococcus, Staphylococcus, Serratia oder Tsukamurella selektiv die Methylgruppe oxidiert.

2. Verfahren gemäß Anspruch 1, dadurch gekennzeichnet, daß man als Bakterium eines aus der Gattung und der Art Micrococcus roseus oder daraus selektionierte Blockmutanten verwendet.

3. Verfahren gemäß Anspruch 1, dadurch gekennzeichnet, daß man als Bakterium eines aus der Gattung und der Art Rhodoccus ruber oder daraus selektionierte Blockmutanten verwendet.

4. Verfahren gemäß Anspruch 1, dadurch gekennzeichnet, daß man als Bakterium Rhodococcus ruber DSM 8316 oder daraus selektionierte Blockmutanten verwendet.

## Claims

1. A process for preparing compounds of the formula I 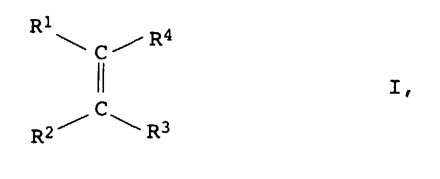 where
R¹ is hydrogen, C₁₋₄-alkyl, C₂₋₄-alkenyl or unsubstituted or substituted aryl,
R² is hydrogen, C₁₋₄-alkyl, C₂₋₄-alkenyl or unsubstituted or substituted aryl, or
R¹ and R² are, together with the carbon atoms to which they are bonded, a) part of an unsubstituted or substituted ring or ring system selected from the group consisting of phenyl, benzoyl, naphthyl, quinolinyl, isoquinolinyl, benzofuryl and benzoxazyl, where the ring or the ring system may be substituted by the following substituents alkyl, alkoxy, alkenyl, halogen, or alkoxycarbonyl, or b) a heterocyclic 5-membered ring which contains 1, 2 or 3 heteroatoms and/or may contain another double bond and to which there may be fused another aromatic or heteroaromatic ring which may be substituted by one to four C₁₋₄-alkoxy, C₁₋₄-alkyl, C₂₋₄-alkenyl, cyano, nitro, carboxyl, C₁₋₄-alkoxycarbonyl, amino, C₁₋₄-alkylamino, di-C₁₋₄-alkylamino and/or an unsubstituted or substituted benzoyl group and/or one to four halogen atoms,
R³ is hydrogen or halogen, C₁₋₄-alkyl or C₂₋₄-alkenyl or unsubstituted or halogen-substituted aryloxy or benzoyl, and
R⁴ is -CH₂OH, -CHO or -COOH,
which comprises the selective oxidation of the methyl group in compounds of the formula II 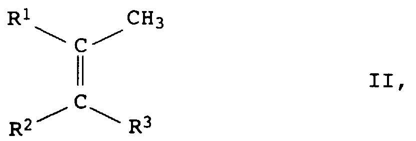 where R¹-R³ have the abovementioned meanings, with the aid of a bacterium selected from the group consisting of the genera Arthrobacter, Bacillus, Brevibacterium, Corynebacterium, Caseobacter, Gordona, Micrococcus, Mycobacterium, Nocardia, Planococcus, Proactinomyces, Rhodococcus, Staphylococcus, Serratia or Tsukamurella.

2. A process as claimed in claim 1, wherein the bacterium used is one from the genus and species Micrococcus roseus or block mutants selected therefrom.

3. A process as claimed in claim 1, wherein the bacterium used is one from the genus and species Rhodococcus ruber or block mutants selected therefrom.

4. A process as claimed in claim 1, wherein the bacterium used is Rhodococcus ruber DSM 8316 or block mutants selected therefrom.

## Revendications

1. Procédé pour la préparaton de composés de formule I 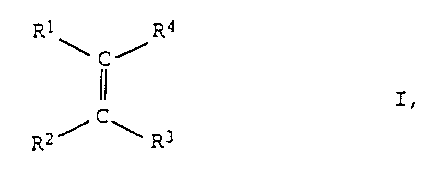 où
R¹ est un atome d'hydrogène, un groupe alkyle en C₁₋₄, un groupe alcényle en C₂₋₄ ou un groupe aryle éventuellement substitué,
R² représente un atome d'hydrogène, un groupe alkyle en C₁₋₄, un groupe alcényle en C₂₋₄ ou un groupe aryle éventuellement substitué, ou
R¹ et R² représentent, conjointement avec les atomes de carbone auxquels ils sont liés, a) une partie d'un cycle ou d'un système cyclique éventuellement substitué, choisi dans le groupe composé de phényle, benzoyle, naphtyle, quinoléinyle, isoquinoléinyle, benzofuryle ou benzoxazyle, tandis que le cycle ou le système cyclique peut être substitué par les substituants suivants alkyle, alcoxy, alcényle, halogène ou alcoxycarbonyle, ou b) un hétérocycle à 5 chaînons, qui contient 1, 2 ou 3 hétéroatomes et/ou peut contenir une autre double liaison et sur lequel peut être condensé un autre cycle aromatique ou hétéroaromatique, qui peut être substitué par un à quatre groupes alcoxy en C₁₋₄, alkyle en C₁₋₄, alcényle en C₂₋₄, nitrile, nitro, carboxyle, alcoxy(C₁₋₄)carbonyle, amino, alkyl(C₁₋₄)amino, dialkyl(C₁₋₄)amino et/ou un groupe benzoyle éventuellement substitué et/ou un à quatre atomes d'halogène,
R³ représente un atome d'hydrogène ou d'halogène, un groupe alkyle en C₁₋₄ ou alcényle en C₂₋₄, ou un groupe aryloxy ou benzoyle éventuellement substitué par des halogènes, et
R⁴ désigne un groupe -CH₂OH ou -CHO ou -COOH, caractérisé par le fait qu'on oxyde sélectivement le groupe méthyle dans des composés de formule II 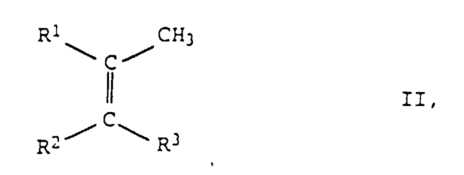
où R¹-R³ possèdent la signification indiquée plus haut, au moyen d'une bactérie choisie dans le groupe des genres Arthrobacter, Bacillus, Brevibacterium, Corynebacterium, Caseobacter, Gordona, Micrococcus, Mycobacterium, Nocardia, Planococcus, Proactinomyces, Rhodococcus, Staphylococcus, Serratia ou Tsukamurella.

2. Procédé selon la revendication 1, caractérisé par le fait qu'on utilise, comme bactéries une bactérie de genre et de type Micrococcus roseus ou des mutants en blocs sélectionnés à partir de celle-ci.

3. Procédé selon la revendication 1, caractérisé par le fait qu'on utilise, comme bactérie, une bactérie de genre et de type Rhodococcus ruber ou des mutants en blocs sélectionnés à partir de celle-ci.

4. Procédé selon la revendication 1, caractérisé par le fait qu'on utilise, comme bactérie, Rhodococcus ruber DSM 8316 ou des mutants en blocs sélectionnés à partir de celle-ci.
